(19) Europäisches Patentamt / European Patent Office / Office européen des brevets

(11) **EP 4 404 832 B1**

(12) **EUROPEAN PATENT SPECIFICATION**

(45) Date of publication and mention of the grant of the patent:
**09.07.2025 Bulletin 2025/28**

(21) Application number: **22790505.6**

(22) Date of filing: **22.09.2022**

(51) International Patent Classification (IPC):
*A61B 5/055* (2006.01)  *A61B 5/00* (2006.01)
*A61B 5/355* (2021.01)  *G01R 33/567* (2006.01)

(52) Cooperative Patent Classification (CPC):
**A61B 5/055; A61B 5/0077; A61B 5/355;**
**A61B 5/7289; G01R 33/5673**

(86) International application number:
**PCT/EP2022/076326**

(87) International publication number:
**WO 2023/046819 (30.03.2023 Gazette 2023/13)**

(54) **SYSTEMS AND METHODS FOR CORRECTING AN ECG SIGNAL IN AN MRI ENVIRONMENT**

SYSTEME UND VERFAHREN ZUR KORREKTUR EINES EKG-SIGNALS IN EINER MRT-UMGEBUNG

SYSTÈMES ET PROCÉDÉS DE CORRECTION D'UN SIGNAL ECG DANS UN ENVIRONNEMENT IRM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.09.2021 US 202163247410 P**

(43) Date of publication of application:
**31.07.2024 Bulletin 2024/31**

(73) Proprietor: **Koninklijke Philips N.V.**
**5656 AG Eindhoven (NL)**

(72) Inventors:
 • **CHACON, Jose H.**
   **5656 AG Eindhoven (NL)**
 • **REDDER, Paul Franz**
   **5656 AG Eindhoven (NL)**
 • **APPLETON, Bruce Geoffrey**
   **5656 AG Eindhoven (NL)**

(74) Representative: **Philips Intellectual Property & Standards**
   **High Tech Campus 52**
   **5656 AG Eindhoven (NL)**

(56) References cited:
**EP-A1- 2 491 858     US-A1- 2008 114 237**

 • **TOBIAS FRAUENRATH ET AL: "Improved Cardiac Triggering by Combining Multiple Physiological Signals: A Cardiac MR Feasibility Study at 7.0 T", INTERNATIONAL SOCIETY FOR MAGNETIC RESONANCE IN MEDICINE, no. 20, 2012, pages 90, XP040575221**
 • **ZU HARTLAGE KAREN MEYER ET AL: "Patient Monitoring During Magnetic Resonance Imaging Exams by Means of Ballistocardiography", 2019 COMPUTING IN CARDIOLOGY (CINC), CREATIVE COMMONS, 8 September 2019 (2019-09-08), XP033720748, DOI: 10.23919/ CINC49843.2019.9005783**

EP 4 404 832 B1

(Cont. next page)

- **SPICHER NICOLAI ET AL: "Initial evaluation of prospective cardiac triggering using photoplethysmography signals recorded with a video camera compared to pulse oximetry and electrocardiography at 7T MRI", BIOMEDICAL ENGINEERING ONLINE, vol. 15, no. 1, 1 December 2016 (2016-12-01), XP055870617, Retrieved from the Internet <URL:https://www. researchgate.net/publication/310780429_Initial_ evaluation_of_prospective_cardiac_triggering_ using_photoplethysmography_signals_recorde d_with_a_video_camera_compared_to_pulse_o ximetry_and_electrocardiography_at_7T_MRI/ fulltext/596e5619a6fdcc2416901294/ Initial-evaluation-of-prospective-car> DOI: 10.1186/s12938-016-0245-3**

## Description

## Field of the Disclosure

The present disclosure is directed generally to systems and methods for correcting an electrocardiogram (ECG) signal impacted by the magnetohydrodynamic effect.

## Background

Magnetic resonant imaging (MRI) scanners utilize magnetic fields, magnetic field gradients, and radio waves to generate a series of images of a patient. In order to focus on the relevant images, the MRI scanner may use a gating or trigger-mechanism. One such mechanism can be an electrocardiogram (ECG). An ECG signal includes a number of waves, segments, complexes, and intervals. In particular, the R-wave is often used to gate MRI image collection, storage, and/or transmission.

In an MRI environment, the gradients produced by the MRI scanner can induce a potential on the vascular system of the patient as their blood circulates, known as the magnetohydrodynamic (MHD) effect. The MHD effect can amplify or distort the T-wave of an ECG signal to the degree that an MRI gating mechanism may confuse the amplified or distorted T-wave for an R-wave. Thus, the MHD can lead to gating errors in MRI gated imaging. Accordingly, there is a need in the art for improved systems for gating MRI image collection based on ECG signals captured in an MRI environment.

United States patent application US20080114237A1 discloses a device for synchronizing a magnetic resonance unit with the cardiac rhythm of a patient, comprising a number of electrodes for leading off electrocardiogram signals from the body of the patient and an evaluation unit for determining a characteristic trigger time within a cardiac rhythm period of the patient from the electrocardiogram signals. The device allows reliable determination of characteristic trigger times within the cardiac rhythm period of the patient even with elevated magnetic field strengths and which operates at least largely independently of external magnetic field by comparing electrocardiogram signals obtained before and after the introduction of the patient into the magnetic resonance unit, in order to determine an enlargement of at least one characteristic of the electrocardiogram signals due to the magnetic field.

European patent application EP2491858A1 discloses a magnetic resonance tomography (MRT) apparatus for the examination of a body comprises parameter acquisition devices for the acquisition of cardiovascular parameters of the body and a control device in communication with the parameter acquisition devices for synchronizing the imaging, wherein the control device is adapted to analyze the data of at least two parameter acquisition devices and to output a control signal based on the analysis.

The journal article Spicher et al. "Initial evaluation of prospective cardiac triggering using photoplethysmography signals recorded with a video camera compared to pulse oximetry and electrocardiography at 7T MRI." Biomedical engineering online 15.1 (2016): 1-28, discloses that accurate synchronization between magnetic resonance imaging data acquisition and a subject's cardiac activity ("triggering") is essential for reducing image artifacts but conventional, contact-based methods for this task are limited by several factors, including preparation time, patient inconvenience, and susceptibility to signal degradation. It further discloses to evaluate the performance of a new contact-free triggering method developed with the aim to eventually replace conventional methods in non-cardiac imaging applications. The method's performance is evaluated in the context of 7 Tesla non-enhanced angiography of the lower extremities.
This journal publication further discloses a basic algorithm capable of estimating in real-time the phase of the cardiac cycle from reflection photoplethysmography signals obtained from skin color variations of the forehead recorded with a video camera. Instead of finding the algorithm's parameters heuristically, they were optimized using videos of the forehead as well as electrocardiography and pulse oximetry signals that were recorded from eight healthy volunteers in and outside the scanner, with and without active radio frequency and gradient coils. Based on the video characteristics, synthetic signals were generated, and the "best available" values of an objective function were determined using mathematical optimization. The performance of the proposed method with optimized algorithm parameters was evaluated by applying it to the recorded videos and comparing the computed triggers to those of contact-based methods. Additionally, the method was evaluated by using its triggers for acquiring images from a healthy volunteer and comparing the result to images obtained using pulse oximetry triggering.

## Summary of the Disclosure

The invention is defined by the independent claims. The dependent claims define advantageous embodiments.

The present disclosure is directed generally to systems and methods for correcting an electrocardiogram (ECG) signal impacted by the magnetohydrodynamic (MHD) effect. The MHD effect occurs due to a patient undergoing magnetic resonant imaging (MRI). The systems and methods derive patient information (such as heart rate) from images captured by a camera, and use this patient information to calculate a T-wave correction factor. This T-wave correction factor is used to counteract the amplification or distortion of the T-wave due to the MHD effect. An MRI scanner may then use the corrected ECG signal for more accurate gated imaging.

The system includes an MRI scanner, an ECG monitor, an MRI-compatible camera, and an image processing unit (IPU). While the patient undergoes an MRI scan, the ECG monitor captures an ECG signal for the patient. At the same time, the camera captures a series of images of the patient. The series of images captured by the camera are provided to the IPU. The IPU processes the images to derive patient information to calculate a T-wave correction factor. The IPU then attenuates the amplitude (or otherwise compensates for the distortion) of the T-wave of the captured ECG signal based on the T-wave correction factor to generate a corrected ECG signal. This corrected ECG signal is then used by the MRI scanner for more accurate gated imaging, as the adjusted T-wave is no longer confused with an R-wave.

Generally, in one aspect, an MRI gating system is provided. The MRI gating system includes an ECG monitor. The ECG monitor is configured to capture an ECG signal of a patient.

The MRI gating system further includes a camera. The camera is configured to capture a series of patient images. According to an example, the camera is a high frame rate camera or a vital signs camera. According to a further example, the camera has a frame rate of at least 1,000 frames per second.

According to an example, the series of patient images capture a neck area of the patient.

The MRI gating system further includes an image processing unit. The image processing unit (IPU) is configured to determine a T-wave correction factor. The T-wave correction factor is determined based on the series of patient images. According to an example, the T-wave correction factor is further based on a patient heart rate derived from the series of patient images.

According to an example, the T-wave correction factor is further based on a diameter of a carotid artery. Further, the T-wave correction factor may be further based on a blood flow velocity in the carotid artery.

According to an example, the T-wave correction factor is further based on a magnetic field incident upon the patient. In this example, the magnetic field may be generated by the MRI scanner.

According to an example, the T-wave correction factor is further based on an angle between a magnetic field incident upon the patient and a direction of the blood flow in the carotid artery.

The IPU is further configured to generate a corrected ECG signal. The corrected ECG signal is generated based on the captured ECG signal and the T-wave correction factor

The MRI gating system further includes an MRI scanner. The MRI scanner is configured to generate a gated MRI image set. The gated MRI image set is generated based on the corrected ECG signal. According to an example, the MRI scanner generates the gated MRI image set further based on one or more R-waves of the corrected ECG signal.

Generally, in another aspect, an MHD effect correction system for an ECG signal is provided. The MHD effect correction system includes a camera configured to capture a series of patient images. The MHD effect correction system further includes an image processing unit configured to (1) determine a T-wave correction factor based on the series of patient images and (2) generate a corrected ECG signal based on the ECG signal and the T-wave correction factor.

Generally, in another aspect, a method for correcting an ECG signal for an MHD effect is provided. The method includes capturing, via a camera, a series of patient images. The method further includes determining, via a processor, a T-wave correction factor based on the series of patient images. The method further includes generating, via the processor, a corrected ECG signal based on a captured ECG signal and the T-wave correction factor. According to an example, the method further includes capturing, via an ECG monitor, the captured ECG signal. According to an example, the method further includes generating, via a magnetic resonating image (MRI) scanner, a gated MRI image set based on the corrected ECG signal.

In various implementations, a processor or controller may be associated with one or more storage media (generically referred to herein as "memory," e.g., volatile and non-volatile computer memory such as RAM, PROM, EPROM, EEPROM, floppy disks, compact disks, optical disks, magnetic tape, SSD, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects as discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the various embodiments will be apparent from and elucidated with reference to the embodiment(s) described hereinafter.

## Brief Description of the Drawings

In the drawings, like reference characters generally refer to the same parts throughout the different views. Also, the drawings are not necessarily to scale, emphasis instead generally being placed upon illustrating the principles of the various embodiments.

FIG. 1 is a system diagram of an magnetic resonant imaging (MRI) gating system, in accordance with an example.

FIG. 2 is an illustration of aspects of an MRI gating system, in accordance with an example.

FIG. 3 is a schematic diagram of an image processing unit (IPU), in accordance with an example.

FIG. 4 is a flow chart of a method for correcting an electrocardiogram (ECG) signal, in accordance with an example.

## Detailed Description of Embodiments

The present disclosure is directed generally to systems and methods for correcting an electrocardiogram (ECG) impacted by the magnetohydrodynamic (MHD) effect. The MHD effect occurs due to a patient undergoing magnetic resonant imaging (MRI). The systems and methods derive patient information (such as heart rate) from images captured by a camera, and use this patient information to calculate a T-wave correction factor. This T-wave correction factor is used to counteract the amplification of the T-wave due to the MHD effect. An MRI scanner may then use the corrected ECG signal for more accurate gated imaging.

The system includes an MRI scanner, an ECG monitor, an MRI-compatible camera, and an image processing unit (IPU). While the patient undergoes an MRI scan, the ECG monitor captures an ECG signal for the patient. At the same time, the camera captures a series of images of the patient. The camera can be a high frame-rate camera or vital signs camera aimed at the neck area of the patient. More specifically, the camera can be aimed at one of the carotid arteries of the patient.

The series of images captured by the camera are provided to the image processing unit. The IPU processes the images to derive patient information to calculate the T-wave correction factor. The IPU can also receive aspects of the patient information from other sources. Prior to deriving the patient information, the image processing unit may stabilize and/or de-noise one or more of the patient images.

First, the IPU determines a heart rate of the patient based on the patient images. The heart rate is determined by analyzing the skin proximate to the carotid artery of the patient. The IPU can use the heart rate of the patient to determine the velocity of the blood flowing through the carotid artery. The IPU also receives and/or derives information regarding the diameter of the carotid artery.

The IPU also receives information regarding the magnetic field generated by the MRI scanner. Specifically, this information can include the magnitude of the magnetic field, as well as the angle between the magnetic field and the direction of blood flow. The magnetic field information can be provided by a sensor, or it can be provided to the IPU directly from the MRI scanner.

The IPU uses the aforementioned information to calculate a T-wave correction factor. The IPU then attenuates the amplitude of the T-wave of the captured ECG signal based on the T-wave correction factor to generate a corrected ECG signal. This corrected ECG signal is then used by the MRI scanner for more accurate gated imaging, as the adjusted T-wave is no longer confused with an R-wave.

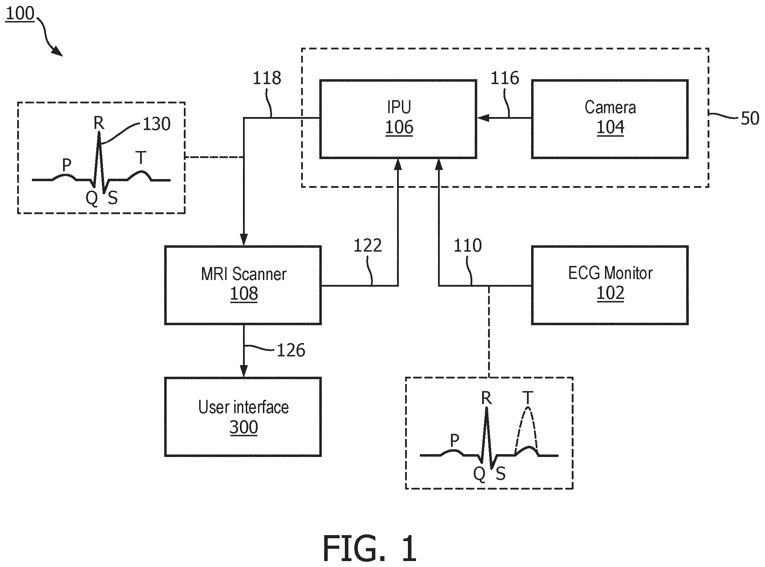

FIG. 1 shows a system diagram for an MRI gating system 100. The MRI gating system 100 generates a gated MRI image set 126 based on a corrected ECG signal 118. The corrected ECG signal 118 is generated by IPU 106 based on patient images 116, captured by camera 104, and an ECG signal 110 captured by an ECG monitor 102.

A patient 200 is inserted into the bore of an MRI scanner 108. In some examples, the entire patient 200 enters the bore; in other examples, only the relevant portion of the patient (head, limb, etc.) enters the bore. The MRI scanner 108 uses magnetic fields, magnetic field gradients, and radio waves to generate a series of MRI images of the patient 200.

While the patient 200 is inserted in the MRI scanner 108 and undergoing MRI scans, ECG monitor 102 captures ECG signals 110 corresponding to the patient 200. The ECG signals 110 may be based on one or more leads connected to one or more electrodes placed on the patient 200. The ECG monitor 102 may be positioned inside or outside of the MRI scanner 108, depending on the application. As depicted in FIG. 1, the ECG signal 110 conveyed from the ECG monitor 102 to the IPU 106 has an amplified T-wave due to the MHD effect.

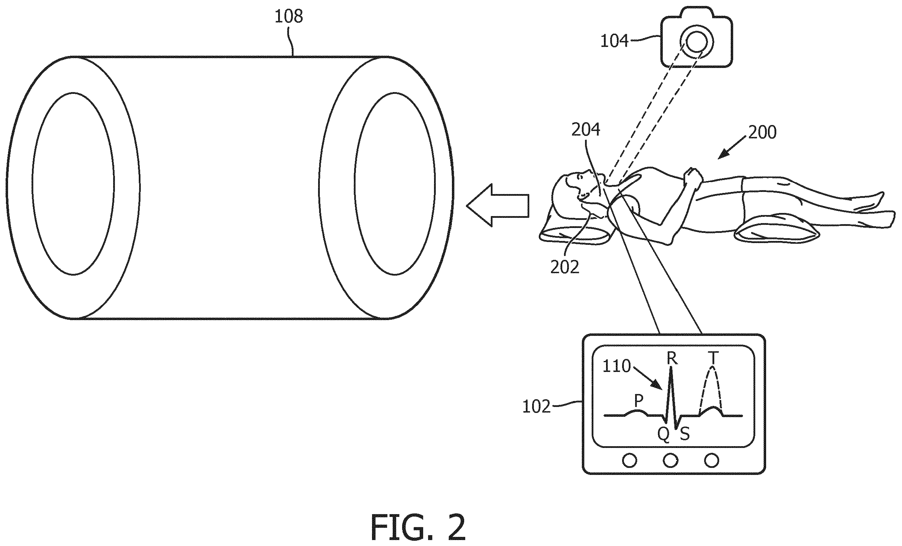

Also, while the patient 200 undergoes MRI scanning, a camera 104, positioned inside the MRI scanner 108, captures a series of patient images 116. The camera 104 may be a high frame rate camera, such as a camera with a frame rate of at least 1,000 frames per second. Alternatively, the camera 104 may be a vital signs camera. As shown in FIG. 2, the camera 104 may be arranged to capture images 116 of the patient's neck area 202, including one of the patient's carotid arteries 204.

The MRI gating system 100 further includes an IPU 106. The IPU 106 is in wired or wireless communication with the MRI scanner 108, the ECG monitor 102, and the camera 104. The IPU receives patient images 116 from the camera 104, ECG signals 110 from the ECG monitor 102, and information regarding the magnetic field 122 from the MRI scanner 108. Alternatively, the information regarding the magnetic field 122 may be captured and conveyed to the IPU 106 via a sensor positioned within the MRI scanner 108. The combination of the IPU 106 and the camera 104 may be considered an MHD effect correction system 50.

The primary purpose of the IPU 106 is to calculate the T-wave correction factor 114 for an ECG signal 110. The T-wave correction factor 114 is applied to the ECG signal 110 to compensate for the amplified T-wave due to the MHD effect. An example T-wave correction algorithm 180 is shown as equation 1 below:

$$U = |B| * |v| * d * sin(q) \quad (1)$$

In equation 1, $U$ is the voltage induced by the MHD effect, $|B|$ is the magnitude 124 of the magnetic field 122 incident upon the patient 200 during MRI scanning, $|v|$ is the blood flow velocity 208 through the carotid artery 204 of the patient 200, $d$ is the diameter 206 of the carotid artery 204, and $q$ is the angle 128 between the lines of the magnetic field 122 and the blood flow direction 210 in the carotid artery 204. In this example, $U$ is the T-wave correction factor 114. Further in this example, $d$ is orthogonal to $B$ and $v$. In other examples, $U$ may be further processed to determine the T-wave correction factor 114. The value of the T-wave correction factor 114 may then be subtracted from the T-wave of the ECG signal 110 to generate the corrected ECG signal 118.

Upon receiving the series of patient images 116, the IPU 108 may pre-process the images 116 using de-noising and/or frame stabilization algorithms 182. Once the images 116 have been pre-processed, the IPU 106 further processes the images 116 to determine the heart rate 120 of the patient 200. The patient heart rate 120 can be determined by analyzing the movement of the patient's skin around one of their carotid arteries 204 via the images 116 captured by a high frame rate camera 104.

The IPU 108 can also use the patient heart rate 120 to determine the blood flow velocity 208. Blood flow velocity 208 may be determined in a number of ways, such as factoring in the patient's blood pressure.

The diameter 206 of the carotid artery 204 may be provided to the IPU 106 by patient records or by a medical professional operating the MRI gating system 100. In further examples, the diameter 206 of the carotid artery 204 can be derived from the patient images 116.

The magnitude 124 of the magnetic field 122 incident upon the patient 200, as well as the angle 128 between the magnetic field 122 and the blood flow direction 208 in the carotid artery, may be provided to the IPU 106 directly by the MRI scanner 108 or via a sensor within the MRI scanner 108.

Once the IPU 106 has either received or determined all of the variables of equation 1, the IPU 106 then determines the T-wave correction factor 114. An example T-wave correction factor 114 may be less than or equal to 1 mV. In one example, the IPU 106 then determines the corrected ECG signal 118 by subtracting the T-wave correction factor 114 from the captured ECG signal 110. In other examples, performs additional calculations with the T-wave correction factor 114 and the captured ECG signal 110 to generate the corrected ECG signal 118. As shown in FIG. 1, the corrected ECG signal 118 has a T-wave of a typical amplitude.

The corrected ECG signal 118 is then provided to the MRI scanner 108 for gated imaging. In gated imaging, the MRI scanner 108 continually generates MRI images, but only stores and/or transmits MRI images corresponding to a gating event or trigger. In one example, the MRI scanner 108 generates one or more gated images 126 based on the R-waves 130 of the corrected ECG signal 118. Accordingly, each of the gated images 126 corresponds to an R-wave 130 of one cycle of the ECG signal 110. Applying the T-wave correction factor 114 to the ECG signal 110 prevents the MRI scanner 108 from confusing the amplified T-waves with R-waves 130, thus preventing gating and/or triggering errors.

In one example, the MRI scanner 108 provides the gated images 126 to a user interface 130 for display. The user interface 130 may be an aspect of the MRI scanner 108 itself, or it may be a component of a stand-alone device, such as a personal computer. The MRI scanner 108 may be in wired or wireless communication with the user interface 130. In a further example, the gated images may be stored in a memory of the MRI scanner and/or a memory of a stand-alone device, such as a personal computer or a network server.

FIG. 2 shows an illustration of some aspects of the MRI gating system, including an MRI scanner 108, camera 104, and ECG monitor 102. The patient 200 is inserted into the bore of the MRI scanner 108 along with the camera 104. While the patient undergoes MRI scanning, the camera 104 captures a series of patient images 112 of the neck area 202 of the patient 200 (which may include one of the patient's carotid arteries 204). Also during the MRI scan, the ECG monitor 102 captures an ECG signal 110. As shown in FIG. 2, this ECG signal 110 has an amplified T-wave during MRI scanning due to the MHD effect.

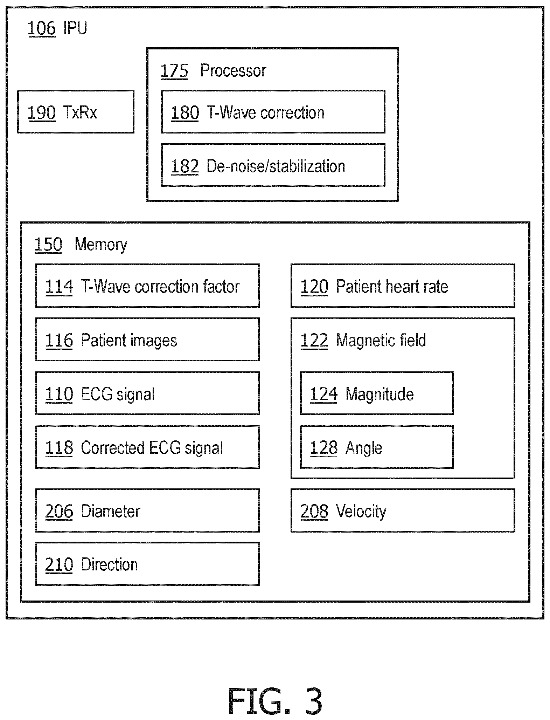

FIG. 3 shows a schematic of the IPU 106. The IPU 106 includes a processor 175 for executing T-wave correction algorithm 180 (such as equation 1), as well as the de-noising and/or frame stabilization algorithms. The IPU 106 further includes memory 150 for storing data received by the other components of the MRI gating system 100 and/or generated by the processor 175. The IPU 106 further includes transceiver 190 for communicating (unidirectionally or bidirectionally) with camera 104, ECG monitor 102, and MRI scanner 108.

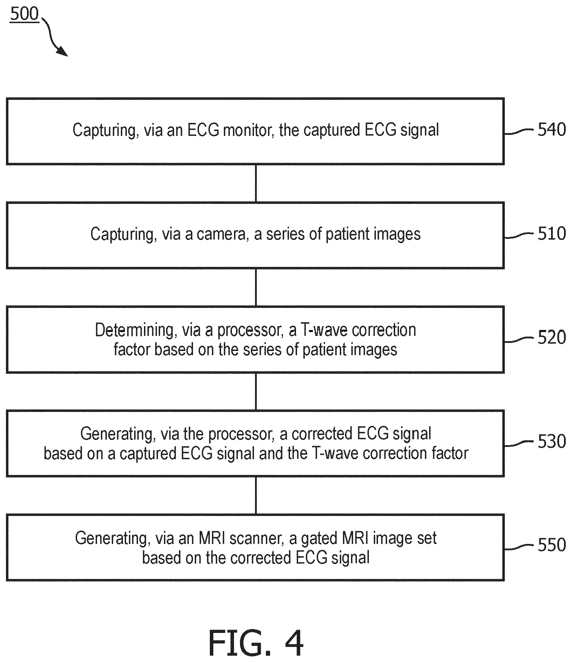

Generally, in another aspect, and with reference to FIG. 4, a method 500 for correcting an ECG signal for an MHD effect is provided. The method 500 includes capturing 510, via a camera, a series of patient images. The method 500 further includes determining 520, via a processor, a T-wave correction factor based on the series of patient images. The method 500 further includes generating 530, via the processor, a corrected ECG signal based on a captured ECG signal and the T-wave correction factor. According to an example, the method 500 further includes capturing 540, via an ECG monitor, the captured ECG signal. According to an example, the method 500 further includes generating 550, via a magnetic resonating image (MRI) scanner, a gated MRI image set based on the corrected ECG signal.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

The above-described examples of the described subject matter can be implemented in any of numerous ways. For example, some aspects may be implemented using hardware, software or a combination thereof. When any aspect is implemented at least in part in software, the software code can be executed on any suitable processor or collection of processors, whether provided in a single device or computer or distributed among multiple devices/computers.

The present disclosure may be implemented as a system, a method, and/or a computer program product at any possible technical detail level of integration. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some examples, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions may be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram or blocks. The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant may be entitled.

While various examples have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the examples described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific examples described herein. It is, therefore, to be understood that the foregoing examples are presented by way of example only and that, within the scope of the patent, examples may be practiced otherwise than as specifically described and claimed.

## Claims

1. A magnetohydrodynamic (MHD) effect correction system (50) for an electrocardiogram (ECG) signal (110), the system being **characterized by** comprising:

    a camera (104) configured to capture a series of patient images (112); and
    an image processing unit (106) configured to:

    determine a T-wave correction factor (114) based on the series of patient images (116); and
    generate a corrected ECG signal (118) based on the ECG signal (110) and the T-wave correction factor (114).

2. A magnetic resonating image (MRI) gating system (100), comprising:

    an electrocardiogram (ECG) monitor (102) configured to capture an ECG signal (110) of a patient (200); and **characterized by**
    the MHD effect correction system (50) according to claim 1 configured to generate a corrected ECG signal (118); and
    an MRI scanner (108) configured to generate a gated MRI image set (126) based on the corrected ECG signal (118).

3. The MRI gating system (100) of claim 2, wherein the camera (104) is a high frame rate camera or a vital signs camera.

4. The MRI gating system (100) of claim 2, wherein the camera (104) has a frame rate of at least 1,000 frames per second.

5. The MRI gating system (100) of claim 2, wherein the T-wave correction factor (114) is further based on a patient heart rate (120) derived from the series of patient images (116).

6. The MRI gating system (100) of claim 2, wherein the T-wave correction factor (114) is further based on a magnitude (124) of a magnetic field (122) incident upon the patient (200).

7. The MRI gating system (100) of claim 6, wherein the magnetic field (122) is generated by the MRI scanner (108).

8. The MRI gating system (100) of claim 2, wherein the series of patient images (116) capture a neck area (202) of the patient (200).

9. The MRI gating system (100) of claim 8, wherein the T-wave correction factor (114) is further based on a diameter (206) of a carotid artery (204).

10. The MRI gating system (100) of claim 9, wherein the T-wave correction factor (114) is further based on a blood flow velocity (208) in the carotid artery (204).

11. The MRI gating system (100) of claim 9, wherein the T-wave correction factor (114) is further based on an angle (128) between a magnetic field (122) incident upon the patient (200) and a blood flow direction (210) in the carotid artery (204).

12. The MRI gating system (100) of claim 2, wherein the MRI scanner (108) generates the gated MRI image set (126) further based on one or more R-waves (130) of the corrected ECG signal (118).

13. A method (500) for correcting an electrocardiogram (ECG) signal for a magnetohydrodynamic (MHD) effect, the method being **characterized by** comprising:

    capturing (510), via a camera, a series of patient images;
    determining (520), via a processor, a T-wave correction factor based on the series of patient images; and
    generating (530), via the processor, a corrected ECG signal based on a captured ECG signal and the T-wave correction factor.

14. The method (500) of claim 13, further comprising capturing (540), via an ECG monitor, the captured ECG signal.

15. The method (500) of claim 13, further comprising generating (550), via a magnetic resonating image (MRI) scanner, a gated MRI image set based on the corrected ECG signal.

## Patentansprüche

1. System (50) zur Korrektur eines magnetohydrodynamischen (MHD) Effekts für ein Elektrokardiogramm (EKG)-Signal (110), wobei das System **dadurch gekennzeichnet ist, dass** es umfasst:

    eine Kamera (104), die so konfiguriert ist, dass sie eine Reihe von Patientenbildern (112) erfasst; und
    eine Bildverarbeitungseinheit (106), die konfiguriert ist zum:

Bestimmen eines T-Wellen-Korrekturfaktors (114) basierend auf der Reihe von Patientenbildern (116); und
Erzeugen eines korrigierten EKG-Signals (118) basierend auf dem EKG-Signal (110) und dem T-Wellen-Korrekturfaktor (114).

2. Magnetresonanzbild-(MRT)-Gatingsystem (100), umfassend:

einen Elektrokardiogramm-(EKG)-Monitor (102), der so konfiguriert ist, dass er ein EKG-Signal (110) eines Patienten (200) erfasst; und
**gekennzeichnet durch**
das MHD-Effekt-Korrektursystem (50) nach Anspruch 1, das so konfiguriert ist, dass es ein korrigiertes EKG-Signal (118) erzeugt; und
ein MRT-Gerät (108), das so konfiguriert ist, dass es basierend auf dem korrigierten EKG-Signal (118) einen Gating-MRT-Bildsatz (126) erzeugt.

3. MRT-Gating-System (100) nach Anspruch 2, wobei die Kamera (104) eine Kamera mit hoher Bildrate oder eine Vitalzeichenkamera ist.

4. MRT-Gating-System (100) nach Anspruch 2, wobei die Kamera (104) eine Bildrate von mindestens 1.000 Bildern pro Sekunde aufweist.

5. MRT-Gating-System (100) nach Anspruch 2, wobei der T-Wellen-Korrekturfaktor (114) weiter auf einer Patientenherzfrequenz (120) basiert, die aus der Reihe von Patientenbildern (116) abgeleitet ist.

6. MRT-Gating-System (100) nach Anspruch 2, wobei der T-Wellen-Korrekturfaktor (114) weiter auf einer Größe (124) eines auf den Patienten (200) einfallenden Magnetfeldes (122) basiert.

7. MRT-Gating-System (100) nach Anspruch 6, wobei das Magnetfeld (122) durch das MRT-Gerät (108) erzeugt wird.

8. MRT-Gating-System (100) nach Anspruch 2, wobei die Reihe von Patientenbildern (116) einen Halsbereich (202) des Patienten (200) erfasst.

9. MRT-Gating-System (100) nach Anspruch 8, wobei der T-Wellen-Korrekturfaktor (114) weiter auf einem Durchmesser (206) einer Halsschlagader (204) basiert.

10. MRT- Gating-System (100) nach Anspruch 9, wobei der T-Wellen-Korrekturfaktor (114) weiter auf einer Blutflussgeschwindigkeit (208) in der Halsschlagader (204) basiert.

11. MRT-Gating-System (100) nach Anspruch 9, wobei der T-Wellen-Korrekturfaktor (114) weiter auf einem Winkel (128) zwischen einem auf den Patienten (200) einfallenden Magnetfeld (122) und einer Blutflussrichtung (210) in der Halsschlagader (204) basiert.

12. MRT-Gating-System (100) nach Anspruch 2, wobei das MRT-Gerät (108) den Gating-MRT-Bildsatz (126) weiter basierend auf einer oder mehreren R-Wellen (130) des korrigierten EKG-Signals (118) erzeugt.

13. Verfahren (500) zum Korrigieren eines Elektrokardiogramm (EKG)-Signals hinsichtlich eines magnetohydrodynamischen (MHD) Effekts, wobei das Verfahren **dadurch gekennzeichnet ist, dass** es Folgendes umfasst:

Erfassen (510) einer Reihe von Patientenbildern mit einer Kamera;
Bestimmen (520) eines T-Wellen-Korrekturfaktors über einen Prozessor, basierend auf der Reihe von Patientenbildern; und
Erzeugen (530) eines korrigierten EKG-Signals über den Prozessor, basierend auf einem erfassten EKG-Signal und dem T-Wellen-Korrekturfaktor.

14. Verfahren (500) nach Anspruch 13, weiter umfassend Erfassen (540) des erfassten EKG-Signals über einen EKG-Monitor.

15. Verfahren (500) nach Anspruch 13, weiter umfassend Erzeugen (550), über ein Magnetresonanzbild(MRT)-Gerät, eines Gating-MRT-Bildsatzes basierend auf dem korrigierten EKG-Signal.

**Revendications**

1. Système de correction d'effet magnétohydrodynamique (MHD) (50) pour un signal d'électrocardiogramme (ECG) (110), le système étant **caractérisé en ce qu'**il comprend :

une caméra (104) configurée pour capturer une série d'images de patient (112) ; et
une unité de traitement d'image (106) configurée pour :

déterminer un facteur de correction d'onde T (114) sur la base de la série d'images de patient (116) ; et
générer un signal ECG corrigé (118) basé sur le signal ECG (110) et le facteur de correction d'onde T (114).

2. Système de synchronisation d'image par résonance magnétique (IRM) (100), comprenant :

   un moniteur d'électrocardiogramme (ECG) (102) configuré pour capturer un signal ECG (110) d'un patient (200) ; et **caractérisé par** le système de correction d'effet MHD (50) selon la revendication 1 configuré pour générer un signal ECG corrigé (118) ; et un scanner IRM (108) configuré pour générer un ensemble d'images IRM synchronisées (126) sur la base du signal ECG corrigé (118).

3. Système de synchronisation IRM (100) selon la revendication 2, dans lequel la caméra (104) est une caméra à fréquence de trame élevée ou une caméra de signes vitaux.

4. Système de synchronisation IRM (100) selon la revendication 2, dans lequel la caméra (104) présente une fréquence de trame d'au moins 1000 trames par seconde.

5. Système de synchronisation IRM (100) selon la revendication 2, dans lequel le facteur de correction d'onde T (114) est en outre basé sur une fréquence cardiaque de patient (120) dérivée de la série d'images de patient (116).

6. Système de synchronisation IRM (100) selon la revendication 2, dans lequel le facteur de correction d'onde T (114) est en outre basé sur une amplitude (124) d'un champ magnétique (122) incident sur le patient (200).

7. Système de synchronisation IRM (100) selon la revendication 6, dans lequel le champ magnétique (122) est généré par le scanner IRM (108).

8. Système de synchronisation IRM (100) selon la revendication 2, dans lequel la série d'images de patient (116) capture une zone de cou (202) du patient (200).

9. Système de synchronisation IRM (100) selon la revendication 8, dans lequel le facteur de correction d'onde T (114) est en outre basé sur un diamètre (206) d'une artère carotide (204).

10. Système de synchronisation IRM (100) selon la revendication 9, dans lequel le facteur de correction d'onde T (114) est en outre basé sur une vitesse de flux sanguin (208) dans l'artère carotide (204).

11. Système de synchronisation IRM (100) selon la revendication 9, dans lequel le facteur de correction d'onde T (114) est en outre basé sur un angle (128) entre un champ magnétique (122) incident sur le patient (200) et une direction de flux sanguin (210) dans l'artère carotide (204).

12. Système de synchronisation IRM (100) selon la revendication 2, dans lequel le scanner IRM (108) génère l'ensemble d'images IRM synchronisées (126) basé en outre sur une ou plusieurs ondes R (130) du signal ECG corrigé (118).

13. Procédé (500) de correction d'un signal d'électrocardiogramme (ECG) pour un effet magnétohydrodynamique (MHD), le procédé étant **caractérisé en ce qu'**il comprend les étapes consistant à :

   capturer (510), par l'intermédiaire d'une caméra, une série d'images de patient ; déterminer (520), par l'intermédiaire d'un processeur, un facteur de correction d'onde T basé sur la série d'images de patient ; et générer (530), par l'intermédiaire du processeur, un signal ECG corrigé basé sur un signal ECG capturé et le facteur de correction d'onde T.

14. Procédé (500) selon la revendication 13, comprenant en outre la capture (540), par l'intermédiaire d'un moniteur ECG, du signal ECG capturé.

15. Procédé (500) selon la revendication 13, comprenant en outre la génération (550), par l'intermédiaire d'un scanner d'image par résonance magnétique (IRM), d'un ensemble d'images IRM synchronisées sur la base du signal ECG corrigé.

FIG. 1

FIG. 2

**106** IPU

**175** Processor

**190** TxRx

**180** T-Wave correction

**182** De-noise/stabilization

**150** Memory

**114** T-Wave correction factor

**120** Patient heart rate

**116** Patient images

**122** Magnetic field

**110** ECG signal

**124** Magnitude

**118** Corrected ECG signal

**128** Angle

**206** Diameter

**208** Velocity

**210** Direction

# FIG. 3

500

Capturing, via an ECG monitor, the captured ECG signal — 540

Capturing, via a camera, a series of patient images — 510

Determining, via a processor, a T-wave correction
factor based on the series of patient images — 520

Generating, via the processor, a corrected ECG signal
based on a captured ECG signal and the T-wave correction factor — 530

Generating, via an MRI scanner, a gated MRI image set
based on the corrected ECG signal — 550

# FIG. 4

## REFERENCES CITED IN THE DESCRIPTION

**Patent documents cited in the description**

- US 20080114237 A1

- EP 2491858 A1

**Non-patent literature cited in the description**

- **SPICHER et al.** Initial evaluation of prospective cardiac triggering using photoplethysmography signals recorded with a video camera compared to pulse oximetry and electrocardiography at 7T MRI.. *Biomedical engineering online*, 2016, vol. 15.1, 1-28